# FASCICULE DE BREVET EUROPEEN

(11) **EP 2 946 806 B1**
(45) Date de publication et mention de la délivrance du brevet: **27.07.2016**
(21) Numéro de dépôt: 15163786.5
(22) Date de dépôt: 16.04.2015
(51) Int. Cl.: A61N 1/36

(54) **DISPOSITIF MÉDICAL IMPLANTABLE ACTIF À OPTIMISATION AUTOMATIQUE DE LA CONFIGURATION D'UNE SONDE DE STIMULATION MULTI-ÉLECTRODE, NOTAMMENT D'UNE SONDE DE STIMULATION SÉLECTIVE DU NERF VAGUE**
AKTIVE IMPLANTIERBARE MEDIZINISCHE VORRICHTUNG MIT AUTOMATISCHER OPTIMIERUNG DER KONFIGURATION EINER MULTIELEKTRODEN-STIMULATIONSSONDE, INSBESONDERE EINER SONDE ZUR SELEKTIVEN STIMULATION DES VAGUSNERVS
ACTIVE IMPLANTABLE MEDICAL DEVICE WITH AUTOMATIC OPTIMISATION OF THE CONFIGURATION OF A MULTI-ELECTRODE STIMULATION PROBE, IN PARTICULAR A PROBE FOR SELECTIVE STIMULATION OF THE VAGUS NERVE

(30) Priorité: 19.05.2014 FR 1454450
(43) Date de publication de la demande: 25.11.2015
(73) Titulaire: Sorin CRM SAS, 92140 Clamart Cedex (FR); INRIA - Institut National de Recherche en Informatique et en Automatique, 78153 Le Chesnay Cedex (FR)
(72) Inventeur: Henry, Chistine, 75014 PARIS (FR); Laporte-Duchemin, Laure, 91420 MORANGIS (FR); Andreu, David, 34570 Montarnaud (FR); Azevedo-Coste, Christine, 34560 Montbazin (FR); Guiraud, David, 34000 Montpellier (FR); Maciejasz, Pawel, 34000 Montpellier (FR); Rossel, Olivier, 34000 Montpellier (FR)
(74) Mandataire: Dupuis-Latour, Dominique

(56) Documents cités:
- WO-A1-2009/020639
- WO-A2-2009/025817
- US-A1- 2012 065 699
- US-A1- 2012 065 702
- US-A1- 2012 330 369
- US-A1- 2014 005 739

## Description

La présente invention concerne d'une façon générale les dispositifs implantables de stimulation nerveuse.

Elle concerne plus particulièrement, mais de façon non limitative, un implant permettant de délivrer des thérapies de stimulation du nerf vague, dites thérapies VNS (*Vagus Nerve Stimulation*)*.*

Un nerf comprend de nombreux axones qui innervent divers organes et muscles du corps humain. Certains de ces axones innervent l'organe, muscle ou structure destinée à faire l'objet de la thérapie, tandis que d'autres innervent des organes, muscles ou structures qui ne sont pas concernés par la thérapie.

Ainsi une stimulation globale, non différenciée, d'un nerf peut au-delà de l'effet thérapeutique désiré, induire des effets indésirables au niveau d'autres organes, muscles ou retours sensoriels. Au surplus, une excitation non différenciée de l'ensemble des fibres d'un nerf, pour avoir l'effet thérapeutique souhaité via le ou les axones concernés, peut nécessiter un courant électrique bien plus élevé que ce qui est nécessaire pour le seul effet thérapeutique recherché.

Il importe donc de délivrer une stimulation spatialement sélective de l'organe cible (typiquement, mais de façon non limitative, un nerf tel que le nerf vague), afin d'aboutir à un effet focalisé sur les paramètres physiologiques visés en limitant les effets indésirables sur des organes ou muscles non visés et en limitant le courant électrique nécessaire pour la stimulation.

La thérapie peut être délivrée de diverses manières - toutes incluses dans le champ de la présente invention - au moyen d'une sonde de neurostimulation disposée autour, à proximité ou à l'intérieur de la structure ciblée.

Dans le cas le plus courant, la sonde est constituée d'un manchon enroulé autour d'un nerf, notamment le nerf vague. Ce manchon est pourvu d'une pluralité d'électrodes venant s'appliquer contre la surface interne du nerf de façon à en stimuler sélectivement certaines régions, par une répartition contrôlée des courants appliqués aux diverses électrodes.

Dans la suite de la description, on fera principalement référence à ce mode de délivrance de la thérapie de stimulation nerveuse, mais on comprendra qu'il ne présente pas de caractère limitatif, l'invention s'appliquant aussi bien à d'autres types de sondes, notamment les sondes tubulaires en forme de stent introduites à l'intérieur d'un vaisseau, par exemple l'aorte, pour stimuler certains sites barorécepteurs ayant un effet indirect sur le système nerveux, ou encore les sondes implantées directement à l'intérieur de l'organe, typiquement un nerf ou le cerveau, pour une stimulation directe, *in situ,* du système nerveux.

On connaît un certain nombre de tentatives pour effectuer une stimulation évoluée, ou pour des applications particulières, de certaines fibres nerveuses. En particulier :
- le US 2012/065702 A1 décrit un dispositif de stimulation à plusieurs électrodes pour des stimulations multiples via celles-ci, avec gestion des priorités en fonction de la réponse motrice ;
- les WO 2009/025817 A2 et WO 2009/020639 A1 décrivent un stimulateur capable d'évaluer la réponse d'un patient à diverses configurations possibles d'électrodes de stimulation, notamment d'électrodes intracardiaques, sur la base de divers critères physiologiques évalués à partir de signaux recueillis par des capteurs, afin de déterminer la configuration procurant la meilleure thérapie ;
- le US 2014/0005739 A1 décrit un neurostimulateur capable d'évaluer la réponse d'un patient à diverses configurations possibles d'électrodes, sur la base d'une analyse du rythme cardiaque de ce patient ;
- l'article de Ordelman et al. "Selectivity for Spécific Cardiovascular Effects of Vagal Nerve Stimulation With a Multi-Contact", IEEE Transactions on Neural Systems and Rehabilitation Engineering, Vol. 21, No. 1, Jan. 2013 enseigne l'application d'une stimulation bipolaire à l'aide de plusieurs paires de contacts sur une électrode gouttière ;
- le WO 2011/040842 A1 décrit un dispositif de stimulation cardiaque dans lequel une série d'électrodes de stimulation sont alimentées par des conducteurs respectifs. Un générateur d'impulsions applique des impulsions sur différentes paires d'électrodes, de façon à exercer des stimulations selon plusieurs modes ;
- la notice # P02216 publiée par le Johns Hopkins University Applied Physics Laboratory, intitulée "Electrode Array to Determine Specific Axonal Firing in a Peripheral Nerve", vise à trouver la fibre à stimuler en examinant les signaux de réponse des différentes fibres ;
- l'article de Tyler et Durand "Functionally Selective Peripheral Nerve Stimulation With a Flat Interface Nerve Electrode", IEEE Transactions on Neural Systems and Rehabilitation Engineering, Vol. 10, No. 4, Dec. 2002 propose une nouvelle géométrie d'électrodes pour une stimulation sélective ;
- les WO 2004/103455 A et WO 03/099377 A enseignent une stimulation unidirectionnelle avec une électrode multipolaire pour une application cardiaque, de façon à neutraliser certains effets induits ;
- le US 2011/0301658 A joue sur différents paramètres de stimulation et différentes positions d'électrodes pour identifier certaines fibres nerveuses pendant la phase chirurgicale ; elle ne concerne pas un dispositif de stimulation autonome implanté ;
- le US 2012/0239109 A1 décrit une configuration quadripolaire d'anodes pour une stimulation épidurale en vue du traitement de la douleur ;
- le US 2012/0065699 A1 décrit une sonde DBS de stimulation cérébrale profonde (*Deep Brain Stimulation*) comportant une multitude d'électrodes de stimulation alimentables de façon indépendante, ainsi que des électrodes de recueil. Il s'agit de pouvoir cibler une aire donnée en jouant sur la configuration de stimulation, notamment pour réduire la consommation du dispositif tout en disposant de moyens de recueillir un signal local ;
- le US 7 483 747 B2 décrit un système de stimulation nerveuse avec optimisation de la consommation et de l'efficacité de l'implant, mais par l'intermédiaire de profils et paramètres de stimulation et non de configurations d'électrodes ;
- le US 2013/0165994 A1 décrit une sonde VNS avec possibilité de commuter d'un jeu d'électrodes à un autre pour maintenir l'efficacité de la stimulation en évitant l'habituation ou en réajustant les cibles après
un déplacement de la sonde VNS.

Toutefois, aucune de ces propositions ne permet une optimisation de la configuration d'électrodes d'un neurostimulateur qui tienne compte à la fois des trois aspects suivants :
- la consommation de l'implant, qui doit être maitrisée de façon très stricte pour ne par obérer la durée de vie de l'implant ;
- la maximisation de l'effet physiologique produit par la thérapie de neurostimulation ; et
- la minimisation des effets secondaires indésirables induits par la stimulation du nerf (par exemple le déclenchement de toux).

Le but de l'invention est de proposer un mode de recherche, de façon entièrement automatique, d'une configuration de stimulation optimale d'une sonde de neurostimulation multi-électrodes qui prenne en compte simultanément ces trois critères.

Par "configuration de stimulation", on entendra toute configuration combinant les critères suivants :
i) le fait qu'une électrode soit ou non une électrode active (c'est-à-dire parcourue ou non par un courant de stimulation) ;
ii) la polarité, anode ou cathode, de chaque électrode active (c'est-à-dire le sens du courant traversant cette électrode) ; et
iii) la répartition entre les différentes électrodes actives du courant produit par le générateur de neurostimulation.

Grâce à l'invention, on pourra ainsi déterminer et figer une configuration d'électrode qui applique la neurostimulation de façon optimale en fonction de l'effet recherché.

À cet effet, l'invention propose un dispositif médical implantable actif de neurostimulation par application contrôlée d'impulsions électriques simultanément en plusieurs points d'un organe, ce dispositif comprenant, de manière en elle-même connue d'après le US 2012/065702 A1 précité : un dispositif de commande pourvu d'un générateur d'impulsions électriques ; une sonde de neurostimulation apte à être placée autour, à proximité ou à l'intérieur de l'organe et comportant un ensemble d'électrodes reliées individuellement au dispositif de commande ; et des moyens de mesure d'un paramètre physiologique représentatif de l'activité cardiaque du patient. Le dispositif de commande comporte un circuit répartiteur associé au dispositif de commande et susceptible de faire varier la configuration de stimulation de manière à stimuler de façon privilégiée certaines régions de l'organe par rapport à d'autres régions. La configuration de stimulation comprend la détermination des électrodes actives reliées au générateur d'impulsions, la polarité anode ou cathode de ces électrodes actives, ainsi qu'éventuellement la répartition des courants respectifs délivrés à ces électrodes actives.

Le dispositif comprend également des moyens de recherche itérative d'une configuration optimale de stimulation, comprenant : des moyens de sélection d'une pluralité de configurations de stimulation différentes ; des moyens de mémorisation du paramètre physiologique mesuré pour chaque configuration de stimulation sélectionnée ; et des moyens de désignation, comme configuration optimale de stimulation, de l'une des configurations de stimulation différentes sélectionnées, en fonction au moins : i) des valeurs mémorisées du paramètre physiologique mesuré pour des configurations d'électrodes différentes.

Le dispositif comprend en outre des moyens de détection d'effets indésirables susceptibles d'être générés par la neurostimulation, les moyens de mémorisation du paramètre physiologique étant également aptes à mémoriser pour chaque configuration de stimulation sélectionnée un indicateur de survenue éventuelle d'un effet indésirable. De plus, la configuration optimale est désignée également en fonction : ii) des valeurs mémorisées de l'indicateur de survenue éventuelle d'un effet indésirable, et iii) du nombre d'électrodes actives utilisées comme cathodes avec chaque configuration de stimulation sélectionnée.

Selon diverses caractéristiques subsidiaires avantageuses :
- la configuration optimale est désignée en fonction du résultat de la comparaison des valeurs mémorisées du paramètre physiologique mesuré avec un seuil-cible prédéterminé ;
- la configuration optimale est désignée également en fonction des valeurs mémorisées du paramètre physiologique mesuré pour différentes répartitions des courants respectifs délivrés aux électrodes, pour une même configuration d'électrodes ;
- les moyens de recherche itérative d'une configuration optimale de stimulation sont également aptes à faire varier au moins un paramètre des impulsions, notamment l'intensité du courant de l'impulsion et/ou le nombre des impulsions d'une salve d'impulsions.

On va maintenant décrire un exemple de mise en oeuvre du dispositif de l'invention, en référence aux dessins annexés où les mêmes références numériques désignent d'une figure à l'autre des éléments identiques ou fonctionnellement semblables. Sur les dessins :
- la Figure 1 est une vue générale de présentation du dispositif de l'invention tel qu'implanté,
- la Figure 2 est une vue plus détaillée d'un ensemble module de commande et sonde de neurostimulation du dispositif de l'invention,
- la Figure 3A est une vue en perspective illustrant un agencement d'électrodes pour un mode de réalisation préféré de la sonde de neurostimulation,
- la Figure 3B illustre un ensemble de configurations possibles de connexions de l'agencement d'électrodes de la Figure 3A,
- les Figures 4a à 4c illustrent qu'une modélisation de la répartition de la stimulation électrique dans la section du nerf pour trois configurations possibles de connexions d'électrodes de la sonde,
- les Figures 5a et 5 b représentent une réponse d'électrocardiogramme en fonction de différentes orientations des configurations d'électrodes des Figures 4 b et 4c,
- la Figure 6 est un organigramme illustrant les différentes étapes d'une procédure de configuration d'un dispositif de neurostimulation implantable selon un premier mode de mise en oeuvre de l'invention,
- la Figure 7 est homologue de la Figure 6, pour un deuxième mode de mise en oeuvre de l'invention, et
- la Figure 8 est homologue de la Figure 6, pour un troisième mode de mise en oeuvre de l'invention.

On va maintenant décrire un exemple de réalisation du dispositif de l'invention.

Les dispositifs de stimulation implantables sont destinés à réaliser une thérapie par neurostimulation (par exemple sur le nerf vague) pour traiter divers symptômes ou états tels qu'une insuffisance cardiaque.

Cette neurostimulation est par exemple réalisée par application d'impulsions électriques à des conducteurs se terminant par des électrodes disposées dans une sonde entourant le nerf (cet exemple particulier n'étant, comme indiqué plus haut, aucunement limitatif de l'invention).

La longévité de la batterie du dispositif implanté dépend des paramètres de stimulation définis par le praticien pour la réalisation de la stimulation dans la durée, principalement l'amplitude, la largeur et la fréquence des impulsions, ainsi que le rapport cyclique de ces impulsions.

En ce qui concerne ses aspects logiciels, l'invention peut être mise en oeuvre par une programmation appropriée du logiciel de commande d'un stimulateur VNS connu. Un tel stimulateur comprend, dans un dispositif de commande implanté, un microprocesseur programmable pourvu de circuits pour mettre en forme et délivrer les impulsions de stimulation aux électrodes implantées. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. Des moyens logiciels participent à la mise en oeuvre de l'invention, étant exécutés par un microcontrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

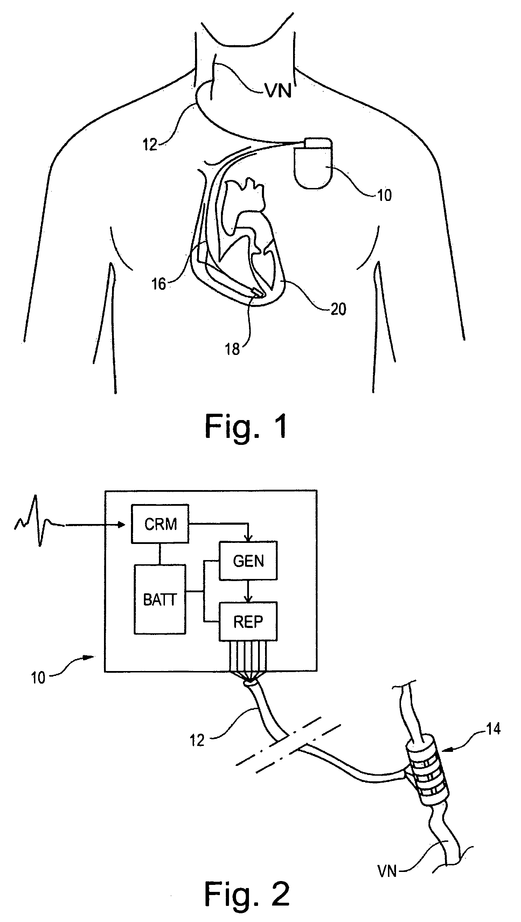

Sur la Figure 1, la référence 10 désigne le dispositif de commande implantable de stimulation du nerf vague. Cette stimulation est délivrée par une sonde 12 portant à sa partie distale 14 un agencement d'électrodes implantées autour du nerf vague VN et susceptibles de stimuler sélectivement certaines fibres de celui-ci par application de salves d'impulsions produites par le générateur 10 sur certaines électrodes comme on le verra en détail dans la suite.

Dans une application particulière au contrôle de l'activité cardiaque, pour permettre la délivrance d'impulsions VNS en synchronisme avec le rythme cardiaque, le générateur 10 dispose également d'une sonde cardiaque 16 pourvue à son extrémité distale d'une électrode 18 de recueil de l'activité électrique du myocarde 20. Cette sonde recueille des signaux d'électrogramme endocavitaire EGM qui permettront de piloter le dispositif de commande 10 afin que celui-ci délivre au nerf vague 14 des impulsions de stimulation VNS au même rythme que les battements cardiaques et au moment le plus approprié de l'onde de dépolarisation cardiaque.

La Figure 2 illustre de façon schématique les principales fonctionnalités du dispositif de commande 10 du dispositif de l'invention.

Celui-ci comprend un circuit générateur GEN apte à produire des salves d'impulsions de neurostimulation destinées à être délivrées au nerf vague via la sonde 12. Dans l'application cardiaque décrite, le circuit générateur GEN est commandé par un circuit CRM de gestion du rythme cardiaque, recevant en entrée le signal EGM en provenance de la sonde 16.

Une première fonction du circuit CRM consiste à piloter le générateur GEN de manière à délivrer des salves d'impulsions de neurostimulation, par exemple en synchronisme avec le rythme cardiaque, ce dernier étant décrit et suivi par des marqueurs correspondant aux instants de survenue de l'onde R, représentative du pic de dépolarisation spontanée des ventricules, de façon connue en soi.

Le dispositif de commande comprend également un circuit de répartition des courants REP permettant de faire varier les configurations de branchements entre le circuit générateur GEN et les électrodes de la sonde 12 du nerf vague, c'est-à-dire que le circuit REP est un circuit apte i) à définir chaque électrode de la sonde 12 comme étant une cathode, ou bien une anode, ou bien une électrode inactive et ii) à répartir le courant produit par le générateur entre les différentes anodes et cathodes ainsi définies. Un tel circuit répartiteur REP est par exemple décrit dans le WO 2006/027473 A1, auquel on pourra se référer pour plus de détails.

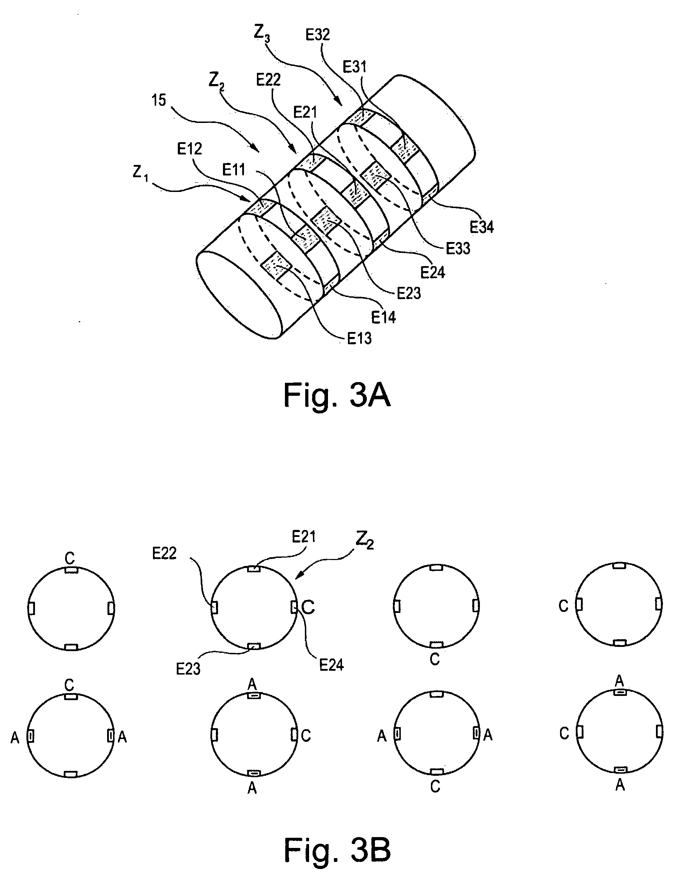

Comme illustré sur la Figure 3A, les électrodes de la sonde 12 sont de préférence agencées selon trois zones annulaires Z1, Z2 et Z3 réparties dans la direction longitudinale du nerf vague et portées par un manchon entourant celui-ci.

Dans une forme de réalisation particulière, toutes les électrodes occupent des secteurs angulaires discrets dans leurs zones annulaires respectives. Par exemple, on prévoit quatre électrodes dans chaque zone annulaire, espacées angulairement de 90° l'une par rapport à la suivante. Ce nombre n'est nullement limitatif. On peut prévoir typiquement entre 2 et 8 électrodes, régulièrement espacées ou non.

Dans les zones annulaires d'extrémités Z1 et Z3, les électrodes sont avantageusement toutes reliées ensemble, pour se comporter de façon analogue, vis-à-vis de la stimulation, à une électrode annulaire d'un seul tenant. En variante, on peut prévoir dans les zones Z1 et Z3 une électrode annulaire généralement continue.

Dans la zone annulaire centrale Z2, les quatre électrodes E21, E22, E23, E24 sont des électrodes de sélectivité connectées individuellement au générateur d'impulsions, via le circuit répartiteur REP, de façon à pouvoir jouer chacune soit le rôle d'anode, soit le rôle de cathode, ou encore à être non connectée (comportement en haute impédance).

Ainsi, la Figure 3B représente huit configurations d'électrodes possibles, où les quatre électrodes E21 à E24 de la zone intermédiaire Z2 sont illustrées avec un rectangle vide désignant une électrode non connectée, tandis qu'un rectangle plein désigne une électrode connectée soit en tant qu'anode (lettre A), soit en tant que cathode (lettre C). On y observe quatre configurations où l'une des électrodes de la zone Z2 est connectée en tant que cathode, et les autres sont non connectées, une configuration parmi celles-ci correspondant à une rotation sur 90° de la précédente. On y observe également quatre configurations où l'une des électrodes est connectée en tant que cathode, ses deux voisines sont connectées en tant qu'anodes, et l'électrode opposée est non connectée. À nouveau, cette configuration est répétée par rotations successives de 90°. Bien entendu, toute autre combinaison de connexions peut être testée.

Dans l'application considérée d'une recherche de l'abaissement du rythme cardiaque, les électrodes des zones terminales Z1 et Z3 jouent typiquement le rôle d'anodes, tandis que les électrodes sectorielles E21 à E24 de la zone intermédiaire Z2 peuvent être non connectées (en haute impédance), connectées en tant que cathodes, ou connectées en tant qu'anodes.

Naturellement, l'entrée de signal du dispositif 10 est adaptée pour que l'unité de commande CRM puisse examiner le signal correspondant à l'effet recherché (ici par exemple un signal d'électrocardiogramme ou un signal de fréquence cardiaque retraité à partir du signal d'électrocardiogramme), et le confronter à une valeur cible à atteindre idéalement lors d'une neurostimulation.

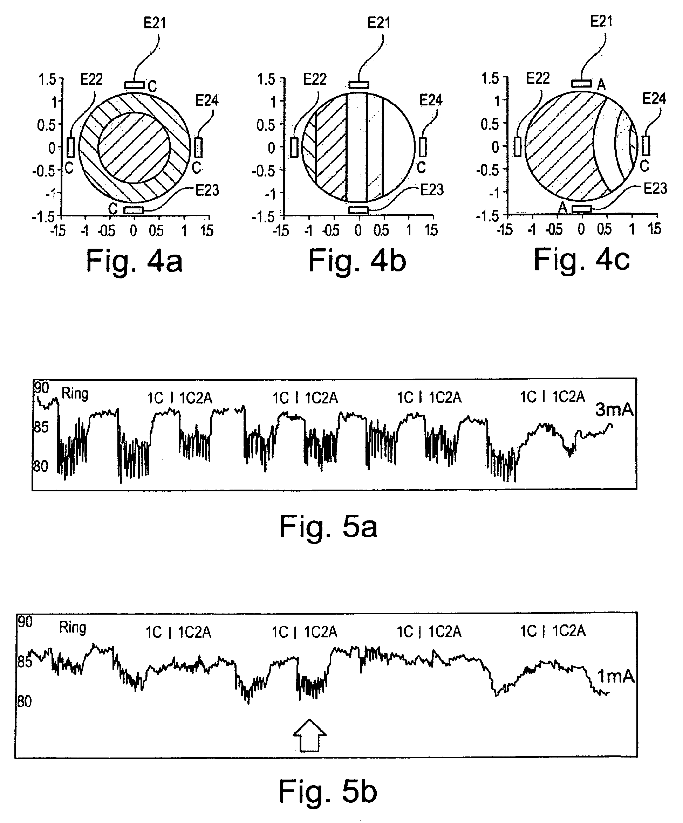

En référence maintenant aux Figures 4a à 4c, on a représenté une simulation des seuils d'activation des fibres avec des configurations différentes d'électrodes. En effet, pour que la stimulation soit la plus sélective possible, il faut que les fibres ciblées aient le seuil d'activation le plus faible possible, et que les fibres non ciblées aient le seuil d'activation le plus fort possible.

La configuration de la Figure 4a correspond au cas où les quatre électrodes E21 à E24 de la zone intermédiaire Z2 sont toutes connectées en tant que cathodes, simulant ainsi le cas connu d'une électrode annulaire. On observe que le seuil d'activation des fibres dans la section du nerf (des seuils différents étant représentés par des hachures de densités plus ou moins fortes) n'est pas différencié angulairement, le seuil étant toutefois légèrement moins élevé en périphérie du nerf que dans sa zone centrale. La configuration de la Figure 4b correspond au cas où trois électrodes de la zone Z2 ne sont pas connectées, et la dernière à droite sur la Figure 4b est connectée en tant que cathode C. On observe dans ce cas un seuil d'activation minimal au voisinage de la cathode, et qui augmente progressivement à mesure que l'on s'écarte horizontalement de la cathode. Enfin, la configuration de la Figure 4c correspond au cas où l'électrode E24 située à droite sur la figure est connectée en tant que cathode, tandis que les deux électrodes adjacentes E21 et E23 (en haut et en bas) sont connectées en tant qu'anodes, c'est-à-dire connectées toutes deux à la zone d'anode Z1 et à la zone d'anode Z3. On observe que la zone de faible seuil d'activation est plus resserrée au voisinage de la cathode, ce qui permet une plus grande sélectivité dans la section du nerf soumise à la stimulation, et donc de cibler plus facilement les fibres nerveuses qui vont être stimulées tout en évitant celles qui ne doivent pas l'être.

La répartition des courants dans ce dernier exemple est par exemple de :
- 25 % dans un sens sur chacune des électrodes Z1, E21, E23 et Z3 (anodes), et 100 % dans l'autre sens sur E24 (cathode).

Mais d'autres répartitions sont envisageables avec le même choix de cathodes/anodes, correspondant donc à des configurations différentes au sens de l'invention, par exemple :
- 20 % dans un sens sur Z1, 30 % sur E21, 25 % sur E23 et 25 % sur Z3 dans le même sens (anodes), et 100 % dans l'autre sens sur E24 (cathode), ou encore
- 40 % dans un sens sur Z1, 10 % sur E21, 10 % sur E23 et 40 % sur Z3 dans le même sens (anodes), et 100 % dans l'autre sens sur E24 (cathode), etc.

Comme on l'a vu plus haut en référence à la Figure 3B, les configurations des Figures 4b et 4c peuvent être tournées de 90° pour donner d'autres configurations qui à leur tour vont permettre de stimuler sélectivement d'autres fibres du nerf.

On comprend que d'autres configurations d'électrodes de la zone Z2 peuvent être également testées, et notamment des configurations avec une cathode et une anode adjacentes, une cathode et une anode opposées, une cathode et trois anodes, etc. ce qui permet de réaliser de multiples combinaisons permettant à chaque fois de stimuler de façon préférentielle une zone de la section du nerf ayant un certain contour. Par ailleurs, il est envisageable non seulement de jouer sur la position des anodes pour focaliser différemment les courants, mais également de mettre en oeuvre plusieurs cathodes (typiquement deux cathodes) pour pouvoir déplacer la zone d'activation et viser ainsi des fibres qui ne seraient pas localisées directement sous une cathode mais plutôt entre deux cathodes.

On comprend également qu'en multipliant le nombre d'électrodes sectorielles dans la zone annulaire Z2 (typiquement jusqu'à 8), on peut définir avec une meilleure résolution la région du nerf stimulée de façon privilégiée.

Il est à noter ici que les zones Z1 et Z3 utilisées comme anodes dans le mode de réalisation qui précède, peuvent également faire l'objet de configurations avec au moins une anode et au moins une électrode non connectée ou une électrode connectée en cathode.

De la sorte, en jouant sur les configurations d'électrodes on peut jouer finement sur la répartition des courants dans la section du nerf, pour ainsi aboutir à une sélectivité dans la stimulation tout en diminuant l'énergie électrique nécessaire à cette stimulation, pour ainsi réduire la longévité de la batterie.

Ainsi peut-on obtenir un effet sur un paramètre physiologique (dans le cas d'espèce, une diminution du rythme cardiaque) qui soit analogue à celui obtenu conventionnellement avec des électrodes annulaires, mais avec un courant de stimulation beaucoup plus limité, et en limitant l'impact indésirable de la stimulation sur d'autres fonctions dans lesquels d'autres fibres du nerf considéré sont impliquées.

La présente invention a pour objet un procédé permettant de déterminer de façon entièrement automatique la configuration optimale pour un patient donné.

Le module de commande CRM du dispositif 10 est apte à piloter le circuit répartiteur REP pour que plusieurs configurations de stimulation soient successivement établies, pendant que le dispositif surveille un signal physiologique particulier censé refléter l'effet souhaité, ceci afin de déterminer et mémoriser la configuration optimale et de figer le circuit de commutation sur cette configuration pour le fonctionnement dans la durée du dispositif implantable.

Il est également possible de répéter le processus de configuration de temps à autre lors des suivis du patient, pour le cas échéant déterminer et mémoriser une autre configuration d'électrodes dont l'effet est meilleur, comme on le verra également dans la suite.

Dans un exemple illustré Figures 5a et 5 b, on a relevé les évolutions de la pulsation cardiaque en fonction de neuf configurations d'électrodes, à savoir à gauche de chaque illustration la configuration de la Figure 4a (notée "RING" car les quatre électrodes autour du nerf sont toutes connectées en tant que cathodes), puis les configurations de la Figure 4 b (notée "1C" pour : une cathode) et de la Figure 4c (notée "1C2A" pour : une cathode et deux anodes), avec les quatre orientations différentes comme mentionné dans ce qui précède et visible sur la Figure 3b.

On observe que pour une intensité d'impulsion de 3 mA (Figure 5a), on ne peut pas identifier une configuration en particulier qui donne une réduction du rythme cardiaque qui soit sensiblement plus importante, en termes d'amplitude et d'homogénéité, que d'autres configurations. En outre, un courant de 3 mA induit immanquablement une diminution de l'autonomie de la batterie du dispositif et de potentiels effets indésirables, en particulier dans une configuration de type RING où une large partie du nerf est stimulée.

En revanche, et comme le montre la Figure 5b, pour une intensité d'impulsions de 1 mA, on observe que pour une certaine configuration de type "1 C2A" (désignée par la flèche), on obtient une réduction significative et homogène du rythme cardiaque, alors qu'une configuration plus standard de type RING (à gauche de la figure) ne permet plus, à ce niveau d'intensité d'impulsion, d'obtenir un effet.

Cette configuration particulière va alors être figée au niveau du dispositif de commutation piloté par le circuit de commande CRM pour que toutes les impulsions, qui dans cet exemple restent déterminées en fonction des signaux reçus de la sonde cardiaque 16, soient appliquées selon cette configuration.

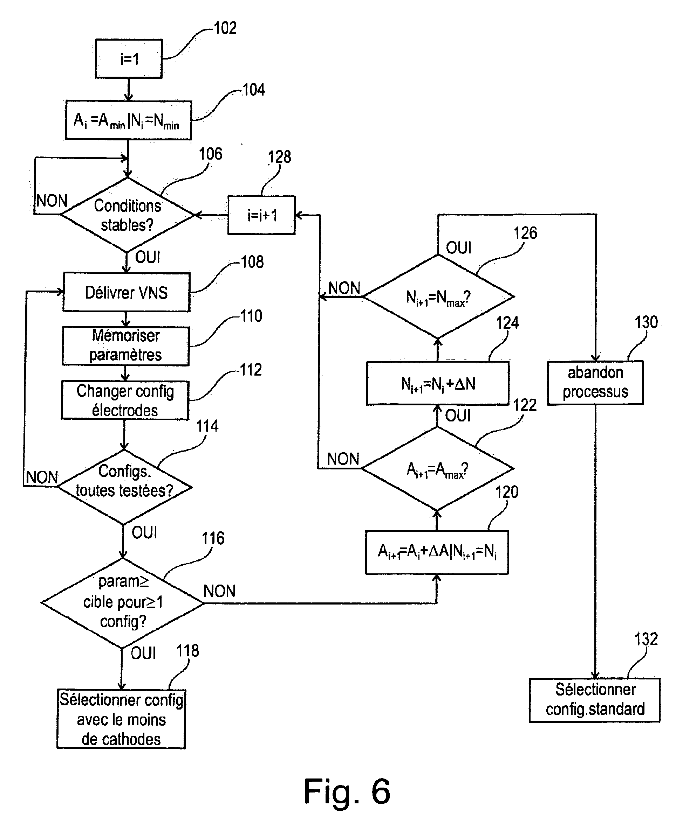

La Figure 6 est un organigramme illustrant les différentes étapes d'une procédure de configuration d'un dispositif implantable de neurostimulation, selon un premier mode de mise en oeuvre de l'invention. Ce processus est le suivant :
- un ou plusieurs paramètres physiologiques (tels que le signal précité d'électrocardiogramme ou un signal de pression dans le ventricule gauche du coeur, ou encore un signal d'accélération endocardiaque) sont enregistrés en utilisant des sondes et un système d'acquisition appropriés, connus en eux-mêmes. Ces signaux sont traités, de façon également connue en soi, pour refléter l'efficacité de la neurostimulation (typiquement un pourcentage d'augmentation de l'intervalle RR donnant le rythme cardiaque, un pourcentage de diminution de la contraction cardiaque, etc.) ;
- une première stimulation est programmée, avec une énergie minimale, soit une amplitude d'impulsions A₁ = Aₘᵢₙ, et un nombre d'impulsions minimal N₁ = Nₘᵢₙ, les autres paramètres étant constants ; par exemple, on fixe A₁ = 1 mA et N₁ = 1 (blocs 102, 104), mais ces valeurs minimales peuvent être préalablement fixées sur d'autres valeurs, par une configuration initiale ;
- si les conditions de stabilité de l'état du patient sont réunies (test du bloc 106), la stimulation telle que programmée est délivrée au nerf par la sonde 12, 14 (bloc 108), et répétée autant de fois qu'il y a de configurations d'électrodes à tester (blocs 112 et 114), après mémorisation des paramètres physiologiques mesurés (bloc 110) et modification de la configuration des électrodes par programmation appropriée du circuit répartiteur REP (bloc 112) ;
- la configuration qui permet d'assurer le meilleur effet cible sur le(s) paramètre(s) physiologique(s) surveillé(s) est déterminée et sélectionnée par le système (blocs 116-118), par exemple en calculant le pourcentage d'augmentation de l'intervalle temporel RR (reflétant une diminution correspondante du rythme cardiaque). En cas de configurations produisant des effets équivalents, celle qui est sélectionnée est celle qui, comprenant le moins de cathodes, consomme le moins d'énergie électrique ;
- dans le cas où l'effet calculé n'atteint pas une valeur cible prédéfinie, des paramètres tels que l'amplitude Aᵢ et/ou le nombre Nᵢ d'impulsions de stimulation sont modifiés (blocs 120-128), et la stimulation est répétée (blocs 110-114) sur les différentes configurations d'électrodes avec ces nouveaux paramètres ;
- si, après avoir essayé toutes les configurations possibles, il n'est pas possible d'atteindre l'effet recherché, alors il est mis fin au processus et une configuration standard de base est sélectionnée par défaut (blocs 130 et 132).

Dans une variante de mise en oeuvre de ce processus, on peut définir une cible prédéterminée (par exemple "réduction de 10 % de la fréquence cardiaque") comme constituant l'optimum et mettre fin au balayage des différentes configurations possibles d'électrodes dès que l'effet cible est atteint, sans attendre d'avoir testé toutes les configurations possibles. Comme on l'a indiqué plus haut, cette procédure de configuration est mise en oeuvre lors de la pose du dispositif implantable de neurostimulation, mais elle peut être également répétée automatiquement de façon périodique, pour le cas échéant déterminer une nouvelle configuration d'électrodes plus efficace. En effet, des phénomènes tels que fibrose, déplacement de la sonde autour du nerf, modification de la réponse du nerf, etc. peuvent amener à ce que la configuration d'électrodes déterminée antérieurement ne soit plus la configuration optimale. Une répétition de la procédure de détermination de la meilleure configuration permet dans ce cas de trouver, puis figer pour une certaine durée jusqu'à la détermination suivante, la nouvelle configuration optimale.

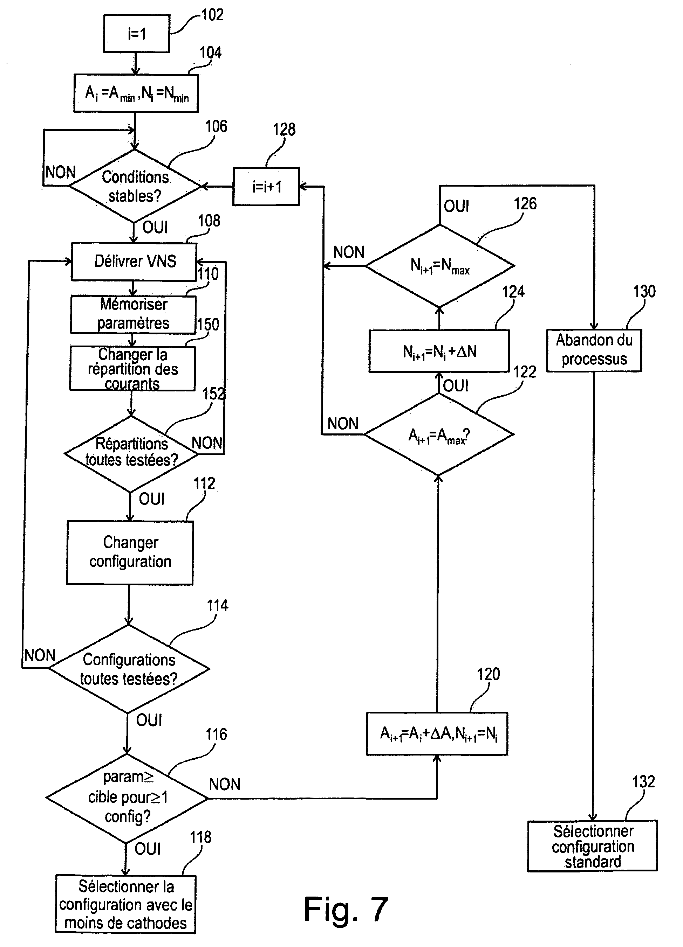

La Figure 7 est un organigramme illustrant les différentes étapes d'une procédure de configuration selon un deuxième mode de mise en oeuvre de l'invention.

Dans cet organigramme, les blocs 102 à 132 correspondent aux mêmes fonctions que celles exposées plus haut dans le cadre de la Figure 6. Le processus diffère en ce qu'il évalue non seulement des configurations d'électrodes différentes (blocs 112 et 114), mais il évalue également, avec une même configuration d'électrodes, l'impact d'une modification de la répartition des courants entre les différentes électrodes (blocs 150 et 152). La sélection de la meilleure configuration de stimulation est donc effectuée sur la base d'un double choix : configuration d'électrodes et répartition des courants.

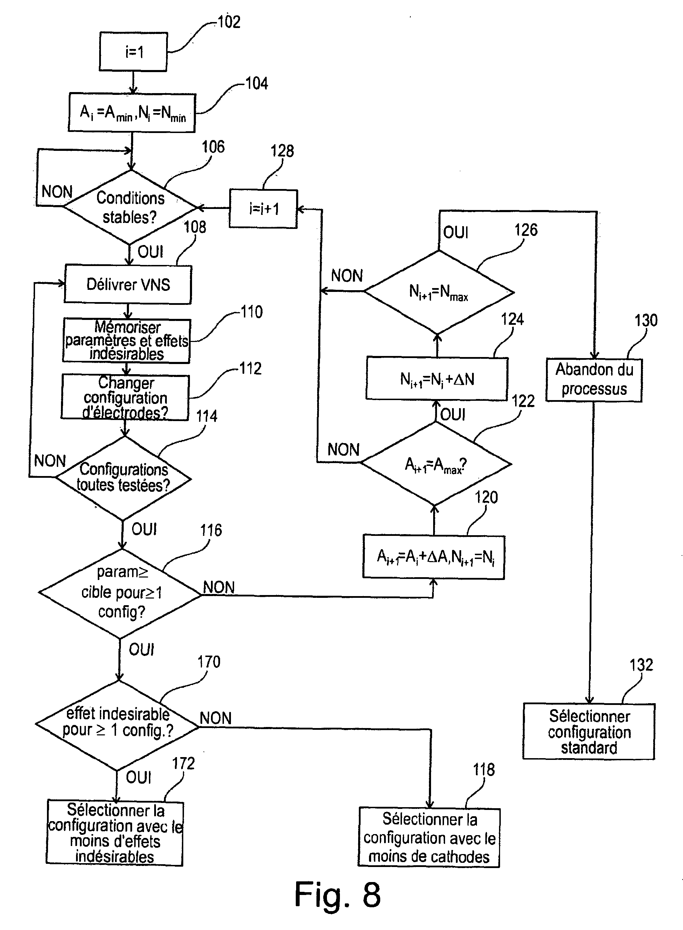

La Figure 8 est un organigramme illustrant les différentes étapes d'une procédure de configuration selon un troisième mode de mise en oeuvre de l'invention.

Ici encore, les blocs 102 à 132 correspondent aux mêmes fonctions que celles exposées plus haut dans le cadre de la Figure 6. Le processus diffère en ce qu'il détecte en outre, simultanément à la mesure du paramètre physiologique (au bloc 110), la survenue éventuelle d'effets indésirables tels que l'apparition de toux, détectées par exemple par analyse d'un signal respiratoire de ventilation-minute ou d'un signal délivré par un accéléromètre. Une fois toutes les configurations testées, celle qui est retenue sera prioritairement celle qui produit le moins d'effets indésirables (blocs 170 et 172).

Bien entendu, la présente invention admet de nombreuses variantes et modifications à la portée de l'homme du métier. En particulier :
- le nombre et l'agencement des électrodes peuvent largement varier, en fonction notamment de la finesse recherchée en matière de sélectivité de la stimulation,
- les calculs destinés à déterminer la meilleure configuration d'électrodes par rapport au but recherché peuvent être effectués soit par le calculateur embarqué dans le dispositif implantable 10, soit par un calculateur externe interfacé de façon appropriée avec le dispositif 10,
- le critère de "meilleure configuration d'électrodes" peut être déterminé très librement en fonction de différents types de signaux, au niveau de différentes fonctions, capables de révéler l'effet d'une neurostimulation, que ce soit sur le nerf vague ou tout autre nerf ayant des fibres associées à différentes fonctions.

L'invention trouve application non seulement dans le domaine de la stimulation et du contrôle de la fonction cardiaque, mais également dans d'autres fonctions préventives ou thérapeutiques telles que la stimulation cérébrale profonde *(Deep Brain Stimulation)* et la stimulation de la moelle épinière, et plus généralement chaque fois qu'une stimulation sélective d'une fonction par certaines fibres d'un nerf est possible.

## Revendications

1. Un dispositif médical implantable actif de neurostimulation par application contrôlée d'impulsions électriques simultanément en plusieurs points d'un organe, comprenant :
- un dispositif de commande (10) pourvu d'un générateur (GEN) d'impulsions électriques ;
- une sonde de neurostimulation (12) apte à être placée autour, à proximité ou à l'intérieur de l'organe (VN), et comportant un ensemble d'électrodes reliées individuellement au dispositif de commande ; et
- des moyens de mesure d'un paramètre physiologique représentatif de l'activité cardiaque du patient,
le dispositif de commande (10) comportant un circuit répartiteur (REP) associé au dispositif de commande et susceptible de faire varier la configuration de stimulation de manière à stimuler de façon privilégiée certaines régions de l'organe par rapport à d'autres régions,
ladite configuration de stimulation comprenant la détermination des électrodes actives reliées au générateur d'impulsions, la polarité anode ou cathode de ces électrodes actives, ainsi qu'éventuellement la répartition des courants respectifs délivrés à ces électrodes actives,
dans lequel le dispositif de commande comprend en outre des moyens de recherche itérative d'une configuration optimale de stimulation, comprenant :
- des moyens de sélection d'une pluraiité de configurations de stimulation différentes ;
- des moyens de mémorisation du paramètre physiologique mesuré pour chaque configuration de stimulation sélectionnée ; et
- des moyens de désignation, comme configuration optimale de stimulation, de l'une desdites configurations de stimulation différentes sélectionnées, en fonction au moins : i) des valeurs mémorisées du paramètre physiologique mesuré pour des configurations d'électrodes différentes,
dispositif comprenant en outre des moyens de détection d'effets indésirables susceptibles d'être générés par la neurostimulation, les moyens de mémorisation du paramètre physiologique étant également aptes à mémoriser pour chaque configuration de stimulation sélectionnée un indicateur de survenue éventuelle d'un effet indésirable, la configuration optimale étant désignée également en fonction :
ii) des valeurs mémorisées de l'indicateur de survenue éventuelle d'un effet indésirable, et
iii) du nombre d'électrodes actives utilisées comme cathodes avec chaque configuration de stimulation sélectionnée.

2. Le dispositif de la revendication 1, dans lequel la configuration optimale est désignée en fonction du résultat de la comparaison des valeurs mémorisées du paramètre physiologique mesuré avec un seuil-cible prédéterminé.

3. Le dispositif de la revendication 1, dans lequel la configuration optimale est désignée également en fonction des valeurs mémorisées du paramètre physiologique mesuré pour différentes répartitions des courants respectifs délivrés aux électrodes, pour une même configuration d'électrodes.

4. Le dispositif de la revendication 1, dans lequel les moyens de recherche itérative d'une configuration optimale de stimulation sont également aptes à faire varier au moins un paramètre des impulsions.

5. Le dispositif selon la revendication 4, dans lequel ledit paramètre des impulsions est l'intensité du courant de l'impulsion et/ou le nombre des impulsions d'une salve d'impulsions.

## Patentansprüche

1. Aktive implantierbare medizinische Vorrichtung zur Neurostimulation durch kontrollierte Anwendung elektrischer Impulse gleichzeitig an mehreren Punkten eines Organs, die enthält:
- eine Steuervorrichtung (10), die mit einem Generator (GEN) elektrischer Impulse versehen ist;
- eine Neurostimulationssonde (12), die um das Organ herum, in der Nähe oder innerhalb des Organs (VN) angeordnet werden kann und eine Einheit von Elektroden aufweist, die einzeln mit der Steuervorrichtung verbunden sind; und
- Einrichtungen zur Messung eines physiologischen Parameters, der für die Herzaktivität des Patienten repräsentativ ist,
wobei die Steuervorrichtung (10) einen Verteilerkreis (REP) aufweist, der der Steuervorrichtung zugeordnet und fähig ist, die Stimulationskonfiguration so zu variieren, dass bestimmte Bereiche des Organs bezüglich anderer Bereiche bevorzugt stimuliert werden,
wobei die Stimulationskonfiguration die Bestimmung der mit dem Impulsgenerator verbundenen aktiven Elektroden, die Anoden- oder Kathodenpolarität dieser aktiven Elektroden sowie ggf. die Verteilung der jeweiligen Ströme enthält, die an diese aktiven Elektroden geliefert werden,
wobei die Steuervorrichtung außerdem Einrichtungen zur iterativen Suche nach einer optimalen Stimulationskonfiguration enthält, die enthalten:
- Einrichtungen zur Auswahl einer Vielzahl unterschiedlicher Stimulationskonfigurationen;
- Einrichtungen zum Speichern des gemessenen physiologischen Parameters für jede ausgewählte Stimulationskonfiguration; und
- Einrichtungen zur Bezeichnung, als optimale Stimulationskonfiguration, einer der verschiedenen Stimulationskonfigurationen abhängig mindestens von:
i) gespeicherten Werten des gemessenen physiologischen Parameters für verschiedene Elektrodenkonfigurationen,
wobei die Vorrichtung außerdem Einrichtungen zur Erfassung unerwünschter Wirkungen enthält, die durch die Neurostimulation erzeugt werden können, wobei die Einrichtungen zum Speichern des physiologischen Parameters ebenfalls für jede ausgewählte Stimulationskonfiguration einen Anzeiger des möglichen Auftretens einer unerwünschten Wirkung speichern können,
wobei die optimale Konfiguration ebenfalls bezeichnet wird abhängig von:
ii) gespeicherten Werten des Anzeigers eines möglichen Auftretens einer unerwünschten Wirkung, und
iii) der Anzahl aktiver Elektroden, die mit jeder ausgewählten Stimulationskonfiguration als Kathoden verwendet werden.

2. Vorrichtung nach Anspruch 1, wobei die optimale Konfiguration abhängig vom Ergebnis des Vergleichs der gespeicherten Werte des gemessenen physiologischen Parameters mit einem vorbestimmten Zielschwellwert bezeichnet wird.

3. Vorrichtung nach Anspruch 1, wobei die optimale Konfiguration ebenfalls abhängig von den gespeicherten Werten des gemessenen physiologischen Parameters für verschiedene Verteilungen der an die Elektroden gelieferten Ströme für die gleiche Elektrodenkonfiguration bezeichnet wird.

4. Vorrichtung nach Anspruch 1, wobei die Einrichtungen zur iterativen Suche einer optimalen Stimulationskonfiguration ebenfalls mindestens einen Parameter der Impulse variieren können.

5. Vorrichtung nach Anspruch 4, wobei der Parameter der Impulse die Stärke des Stroms des Impulses und/oder die Anzahl der Impulse eines Impulszugs ist.

## Claims

1. An active implantable medical device for neurostimulation by controlled application of electrical pulses simultaneously to several points of an organ, comprising:
- a control device (10) provided with a generator (GEN) of electric pulses;
- a neurostimulation probe (12) adapted to be placed around, near or inside the organ (VN), and including a set of electrodes individually connected to the control device; and
- means for measuring a physiological parameter representative of the cardiac activity of the patient,
the control device (10) including a distributor circuit (REP) associated with the control device and liable to vary the stimulation configuration so as to stimulate in a privileged manner certain regions of the organ with respect to other regions,
said stimulation configuration comprising the determination of the active electrodes connected to the pulse generator, the anode or cathode polarity of these active electrodes, as well as possibly the distribution of the respective currents delivered to these active electrodes,
the control device further comprising means for iteratively searching for an optimal stimulation configuration, comprising :
- means for selecting a plurality of different stimulation configurations;
- means for memorizing the physiological parameter measured for each stimulation configuration selected; and
- means for designating, as an optimum stimulation configuration, one of said different stimulation configurations selected, as a function at least of: i) the memorized values of the physiological parameter measured for different configurations of electrode,
the device further comprising means for detecting undesirable effects liable to be generated by the neurostimulation, the means for memorizing the physiological parameter being also adapted to memorize for each stimulation configuration selected an indicator of potential occurrence of an undesirable effect, the optimum configuration being also designated as a function of:
ii) the memorized values of the indicator of potential occurrence of an undesirable effect, and
iii) the number of active electrodes used as cathodes with each stimulation configuration selected.

2. The device of claim 1, wherein the optimum configuration is designated as a function of the result of the comparison of the memorized values of the measured physiological parameter with a predetermined target threshold.

3. The device of claim 1, wherein the optimum configuration is also designated as a function of the memorized values of the measured physiological parameter for different distributions of the respective currents delivered to the electrodes, for a same configuration of electrodes.

4. The device of claim 1, wherein the means for iteratively searching for an optimum stimulation configuration are also adapted to vary at least one parameter of the pulses.

5. The device of claim 4, wherein said parameter of the pulses is the current intensity of the pulse and/or the number of pulses of a pulse burst.
